# EUROPEAN PATENT SPECIFICATION

(11) **EP 4 248 912 B1**
(45) Date of publication and mention of the grant of the patent: **22.10.2025**
(21) Application number: 22163217.7
(22) Date of filing: 21.03.2022
(51) Int. Cl.: A61F 2/08

(54) **GRAFT SUSPENSION DEVICE AND ARRANGEMENT**
TRANSPLANTATAUFHÄNGUNGSVORRICHTUNG UND -ANORDNUNG
DISPOSITIF ET AGENCEMENT DE SUSPENSION DE GREFFE

(43) Date of publication of application: 27.09.2023
(73) Proprietor: Inion Oy, 33520 Tampere (FI)
(72) Inventor: Heinonen, Tuomas, 33560 Tampere (FI); Vuorisalo, Vesa, 33530 Tampere (FI); Mäenpää, Susanna, 33580 Tampere (FI); Heino, Harri, 33340 Tampere (FI)
(74) Representative: Papula Oy

(56) References cited:
- EP-A2- 2 676 613
- WO-A1-2007/109665
- CN-B- 108 209 990
- US-A1- 2015 025 631

## Description

### BACKGROUND

The invention relates to a graft suspension device.

The invention further relates to a graft suspension arrangement.

Surgical buttons are one type of graft suspension devices that are implants used for fixing the ligaments into channels drilled in bone tissue in several surgical reconstruction surgeries. Typical application areas are anterior and posterior ligaments of the knee, syndesmosis ligament healing support in an ankle and in acromioclavicular joint fixation in shoulder. The load bearing requirement for ACL and PCL fixation of an adult knee may be e.g. 500 N.

A problem with known surgical button -type of graft suspension devices is that their design does not provide enough strength if manufactured from polymer materials.

### BRIEF DESCRIPTION

Viewed from a first aspect, there can be provided a graft suspension device, comprising an elongate body having a first end, a second end, and a longitudinal axis extending therebetween, a ridge arranged on the longitudinal axis, on a first side of the graft suspension device, a first side routing surface arranged next to a first side of the ridge, a second side routing surface arranged next to a second side of the ridge, so that the ridge lies between the first and second routing surfaces when seeing from direction of the first side, and wherein the first and second routing surfaces are extending from the first side to a second side of the device.

Thereby a graft suspension device the design of which allows the use of lower strength materials than before, opening possibilities for strong bioabsorbable materials like magnesium alloys and mineral fiber reinforced polymer composites, may be achieved.

Viewed from a further aspect, there can be provided a graft suspension arrangement, comprising the graft suspension device as defined in this disclosure, and a loop, a pulling suture, and optionally a flipping suture.

Thereby it is possible to achieve a graft suspension arrangement where use of metals is not necessary.

The graft suspension device and the arrangement are characterised by what is stated in the independent claims. Some other embodiments are characterised by what is stated in the other claims. Inventive embodiments are also disclosed in the specification and drawings of this patent application. The inventive content of the patent application may also be defined in other ways than defined in the following claims. The inventive content may also be formed of several separate inventions, especially if the invention is examined in the light of expressed or implicit sub-tasks or in view of obtained benefits or benefit groups. Some of the definitions contained in the following claims may then be unnecessary in view of the separate inventive ideas. Features of the different embodiments of the invention may, within the scope of the basic inventive idea, be applied to other embodiments.

According to the invention, at least one of the first side routing surface and the second side routing surface is arranged in a groove that is arranged to constitute a part of a respective sidewall of the device.

An advantage is that a closed loop can be attached to the device.

According to the invention, at least one of the first side routing surface and the second side routing surface is arranged in a hole.

An advantage is that the loop may be secured to the device.

In one embodiment, said groove or said hole has an elongated, such as oval, cross-section.

An advantage is that the elongated shape provides cross-sectional area enough for receiving the loop, while limiting the width and strength of the ridge as little as possible.

In one embodiment, the device comprises at least one projection extending sideways away from the ridge, and wherein at least one of the first side routing surface and the second side routing surface is arranged on the surface of the at least one projection.

An advantage is that a simple structure for supporting and guiding the loop may be achieved.

In one embodiment, the side routing surfaces are arranged asymmetrically so that one of the side routing surfaces is located closer to the first end than another side routing surface.

An advantage is that the device may slide in a drilled channel easier since the loop's sides are positioned in different spots in the lengthwise direction of the device.

In one embodiment, the device comprises a first through hole extending through the device and arranged closer to the second end than to the first end.

An advantage is that a flipping suture may be placed optimally to the device.

In one embodiment, the device comprises a holding surface comprising a concave shape, the holding surface being arranged closer to the first end than to the second end.

An advantage is that the holding surface establishes a simple means for keeping the loop at a correct place during implantation operation.

In one embodiment, the holding surface is arranged in a second through hole.

An advantage is that the loop may be secured to the device.

In one embodiment, the second through hole is arranged to extend from the first side of the device to the second side of the device.

An advantage is that a suture passed through the second through hole may be used for holding the loop in a particular place during implantation operation.

In one embodiment, the device comprises a plateau arranged between the ridge and the first end on the first side of the graft suspension device, the plateau joining to the side routing surfaces, and wherein the second through hole is arranged in the plateau.

An advantage is that the plateau creates a specific place to keep the loop away from the middle section of the device, thus enabling high material thickness and strength in said middle section.

In one embodiment, the device comprises an end wall arranged at the first end, the end wall bordering the plateau.

An advantage is that positioning of the loop on the plateau during the device is sliding in the drilled channel may be enhanced.

In one embodiment, the ridge has a sloping surface from the plateau on the ridge.

An advantage is that as the device is turning to a securing position where the the graft suspension device is flat across the channel, the loop moves easily from the plateau on the ridge.

In one embodiment, the device comprises a middle bulge arranged in an area between the first and the second side routing surfaces on the second side of the device.

An advantage is that the height of the middle section of the device can be extended and thus the strength of said middle section is high.

In one embodiment, the length of the ridge exceeds the width of the device.

An advantage is that a load bearing beam having high strength may be created across the drilled channel.

### BRIEF DESCRIPTION OF FIGURES

Some embodiments illustrating the present disclosure are described in more detail in the attached drawings, in which
Figures 1a - 1c are schematic views of a graft suspension device,
Figures 2a - 2c are schematic views of another graft suspension device,
Figures 3a, 3b are schematic views of a third graft suspension device,
Figures 4a - 4c are schematic views of a fourth graft suspension device,
Figures 5a, 5b are schematic views of a fifth graft suspension device, and
Figures 6a- 6c are schematic views of a graft suspension arrangement.

In the figures, some embodiments are shown simplified for the sake of clarity. Similar parts are marked with the same reference numbers in the figures.

### DETAILED DESCRIPTION

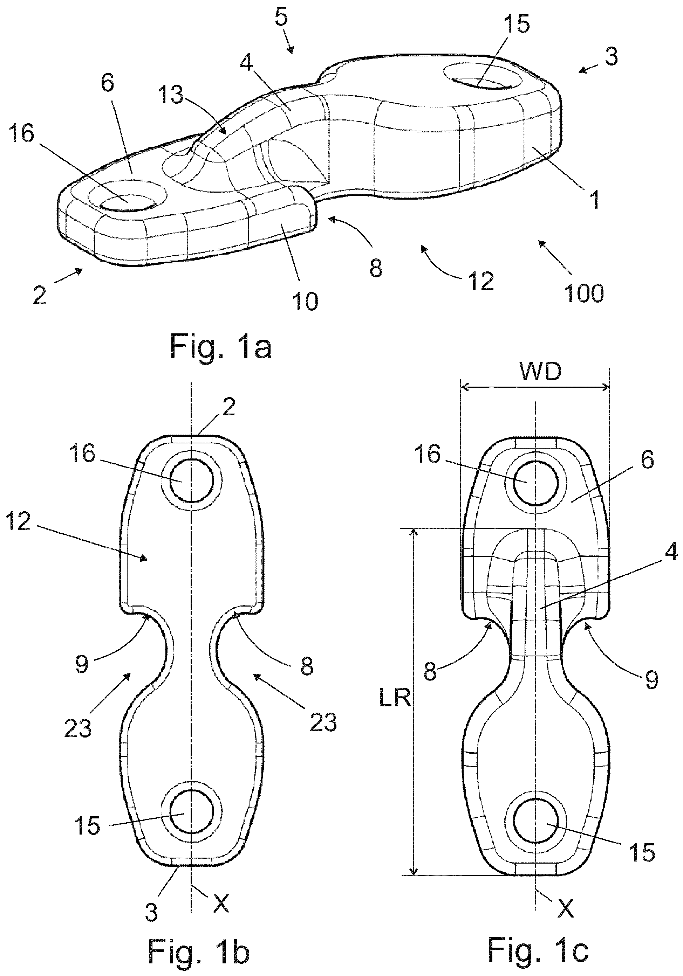

**Figures 1a - 1c** are schematic views of a graft suspension device. It is to be noted that the graft suspension devices described in this disclosure are embodiments of surgical buttons.

The graft suspension device 100 comprises an elongate body 1 having a first end 2 and a second end 3. A longitudinal axis X of the device 100 extends between said ends 2, 3.

Furthermore, the device 100 has a first side or side surface 5 and a second side or side surface 12. The imaginary longitudinal axis X connects the center of the first end 2 and the center of the second end 3.

A ridge 4 is arranged on the longitudinal axis, on the first side 5 of the graft suspension device. In one embodiment, the graft suspension device 100 is dimensioned so that the length LR of the ridge exceeds the width WD of the device.

On a first side of the ridge 4 there is a first side routing surface 8, whereas on a second side of the ridge there is a second side routing surface 9. The ridge thus lies between the side routing surfaces 8, 9 if the device is observed from direction of the first side 5. The first and second routing surfaces 8, 9 extend from the first side 5 to the second side 12 of the device. The side routing surface 8, 9 preferably curves smoothly from the first side 5 to the second side 12, i.e. preferably there are no sharp-cornered edges in the surface.

In one embodiment, the side routing surface 8, 9 is a part or a section of a groove 23. The groove 23 is a part of the respective sidewall 10, 11 of the device. The grooves may have at least essentially equal shapes and sizes, but this is not necessary, i.e. in some another embodiment there may be size and/or shape variations between the grooves.

In one embodiment, the grooves 23 have an elongated (in direction of the longitudinal axis X) shape, such as an oval, shape.

In one embodiment, such as in the embodiment shown in Figures 1a-1c, the grooves 23 side are arranged symmetrically in the device such that both routing surfaces 8, 9 have a same distance to the first end of the device.

The side routing surfaces 8, 9 are intended for routing a loop described later in this disclosure.

The graft suspension device 100 comprises a first through hole 15 that is arranged closer to the second end 3 than to the first end 2. The first through hole 15 extends through the device. In one embodiment, such as in the embodiment shown in Figures 1a-1c, the first through hole 15 extends from the first side 5 to the second side 12 of the device, and is arranged symmetrically in relation to the longitudinal axis X.

In one embodiment, the first through hole 15 is intended for routing a flipping suture described later in this disclosure.

The graft suspension device 100 further comprises a second through hole 16 that is arranged closer to the first end 2 than to the second end 3. In one embodiment, the second through hole 16 is intended for routing a pulling suture described later in this disclosure.

In one embodiment, the second through hole 16 extends from the first side 5 of the device to the second side 12 of the device.

In one embodiment, the second through hole 16 is a round hole arranged so that the longitudinal axis X cuts the center of thereof.

The graft suspension device 100 may comprise a plateau 6 that is arranged between the ridge 4 and the first end 2 on the first side 5 of the graft suspension device, and the second through hole 16 is arranged in the plateau. In one embodiment, the plateau 6 comprises at least substantially flat surface that joins smoothly to the side routing surfaces 8, 9.

It will be appreciated that, by having open grooves 23 combined with the plateau 6, a closed loop can be located after manufacture of the device 100. Alternatively, the loop can be manufactured in situ.

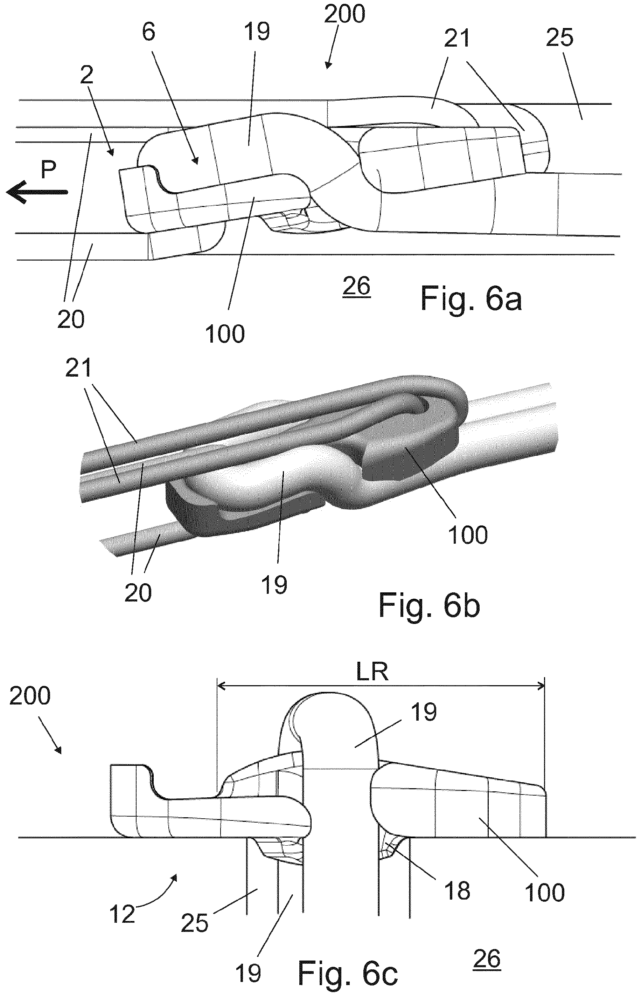

In one embodiment, the ridge 4 comprises a sloping surface 13 that joins to the plateau 6 and rises towards the top 14 of the ridge 4. The sloping surface 13 is preferably without any point of discontinuity or other shapes that could hamper the loop (shown in Figures 6a-6b) sliding over.

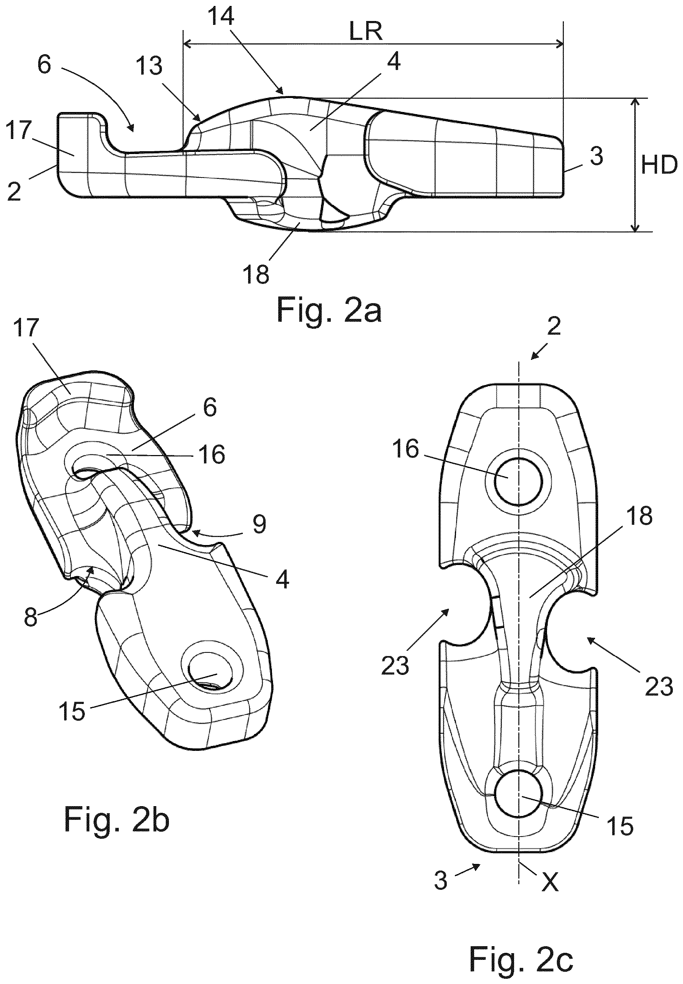

**Figures 2a - 2c** are schematic views of another graft suspension device. The graft suspension device 100 comprises an elongate body 1 having a first end 2 and a second end 3. A longitudinal axis X of the device 100 extends between said ends 2, 3. The device 100 has a first side surface 5 and a second side surface 12, too.

A ridge 4 is arranged on the longitudinal axis on the first side 5 of the graft suspension device. The length LR of the ridge exceeds the width WD of the device. Typically, the width WD is maximized in relation to the diameter of the drilled channel and thus, the ridge longer than the width WD may create a load carrying beam over the channel when the device is in a securing position, such as shown in Figure 6c.

In one embodiment, the ridge 4 has a convex shaped profile having a top 14, i.e. a distinct culmination point of the profile.

In one embodiment, such as in the embodiment shown in Figures 2a-2c, the device 100 comprises two grooves 23 arranged on sides of the ridge 4. The grooves 23 constitutes a part of a respective sidewall 10, 11 of the device. The shape of the groove 23 may be selected rather freely. In one embodiment, the groove 23 has an elongated shape, such as oval or oviform.

In one embodiment, the side of the groove 23 is narrower than the rest of the groove so that probability of slipping the loop out from the groove may be lessened.

The first of said grooves 23 comprises a first side routing surface 8 whereas another of said grooves 23 comprises a second side routing surface 9. The routing surfaces 8, 9 are connected on a plateau 6 between the ridge 4 and the first end 2 on the first side 5 of the graft suspension device.

In one embodiment, the side routing surfaces 8, 9 are arranged asymmetrically (best shown in Figure 2c) so that one of the side routing surfaces is located closer to the first end 2 than another side routing surface.

In one embodiment, a plateau 6 lies between the ridge 4 and the first end 2 of the graft suspension device. The plateau 6 joins to the side routing surfaces 8, 9 smoothly and continuous way. A second through hole 16 extending from the first side 5 to the second side 12 of the device is arranged in the plateau 6.

It will be appreciated that, by having open grooves 23 combined with the plateau 6, a closed loop can be located after manufacture of the device 100. Alternatively, the loop can be manufactured in situ.

In one embodiment, the ridge 4 has a sloping surface 13 that rises from the plateau 6. In one embodiment, the second through hole 16 is placed at the root of said sloping surface 13.

In one embodiment, an end wall 17 is arranged at the first end 2 for bordering the plateau 6. In one embodiment, The height of the end wall 17 measured from the plateau is at least half of the loop used in the graft suspension arrangement described in this disclosure. The end wall 17 creates together with the plateau 6 a space securing and shielding the loop or bend thereof when pulled through a channel, such as shown in Figure 6a.

A first through hole 15 is arranged to an opposite half of the device compared to the second through hole 16. In one embodiment, the first through hole 15 extends through the device from the first side 5 to the second side 12 of the device, symmetrically in respect of the longitudinal axis X.

In one embodiment, such as in the embodiment shown in Figures 2a-2c, both the first and the second through holes 15, 16 are extending from the first side 5 to the second side 12 of the device.

In one embodiment, a middle bulge 18 is arranged on the second side 12 of the device and in an area between the first and the second side routing surfaces 8, 9. In one embodiment, the height HD of the device is defined by the ridge 4 and the middle bulge 18. In one embodiment, the height HD is less than the width WD of the device. In one embodiment, the height HD is same or at least substantially same as the width WD of the device.

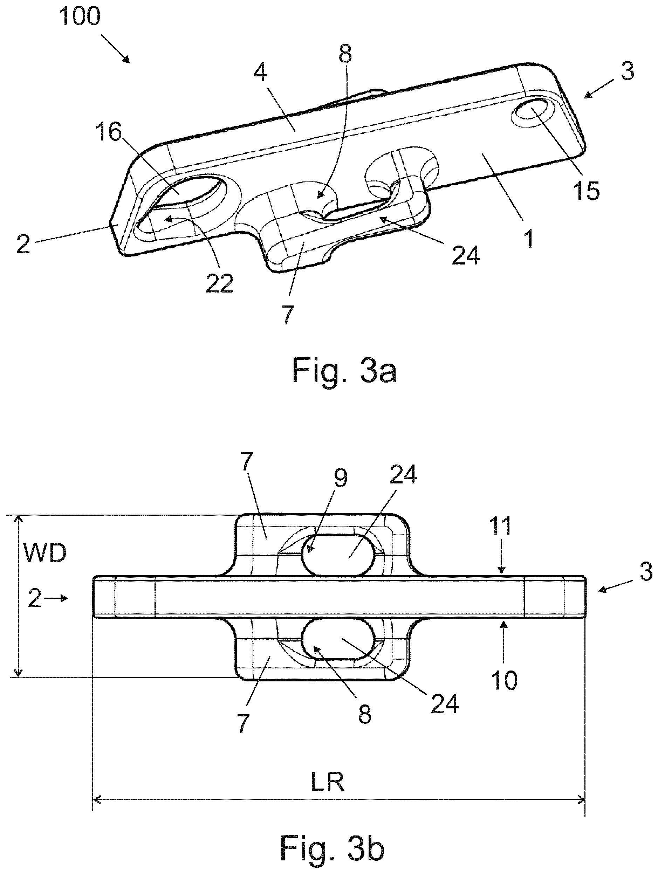

**Figures 3a, 3b** are schematic views of a third graft suspension device. In this embodiment, the body 1 of the device has a box-like middle-section to sides of which two projections 7 are arranged. The box-like middle-section establishes a ridge 4, or in another words, the middle-section and the ridge are fully integrated. Said projections 7 extend sideways away from the middle-section and the ridge 4.

In one embodiment, the first and second side routing surfaces 8, 9 are arranged on the surfaces of the projections 7. In one embodiment, at least one of the first and second side routing surfaces 8, 9 are arranged in a hole 24. In the embodiment shown in Figures 3a-3b, both the first and second side routing surfaces 8, 9 are arranged in a hole 24. Said hole may have an elongated, such as oval or oviform, or a round cross-section, for instance.

In one embodiment, a first through hole 15 arranged closer to the second end 3 than to the first end 2 extends through the body in direction that is aligned more with the width WD than with the height of the device, or in other words, from a first sidewall 10 to a second sidewall 11 of the device.

In one embodiment, a second through hole 16 arranged closer to the first end 2 than to the second end 3 extends through the body in direction that is aligned more with the width WD than with the height of the device, i.e. from a first sidewall 10 to a second sidewall 11 of the device.

In one embodiment, such as in the embodiment shown in Figures 3a-3b, both the first and the second through holes 15, 16 are extending from the first sidewall 10 to the second sidewall 11 of the device.

In one embodiment, the device 100 comprises a holding surface 22 that is utilized as a means for keeping a loop in its intended place during operation. In one embodiment, such as in the embodiment shown in Figures 3a-3b, the holding surface 22 is arranged in the second through hole 16 arranged closer to the first end 2 than to the second end 3. Therefore, the size and shape of said second through hole 16 is selected so that the loop can be inserted therethrough easily. One embodiment of the loop is shown in Figures 6a-6b.

It will be appreciated that, by having the holes 24 and the holding surface 22 in the second through hole 22, a closed loop can be located in manufacturing of the device 100. Alternatively, the loop can be manufactured in situ.

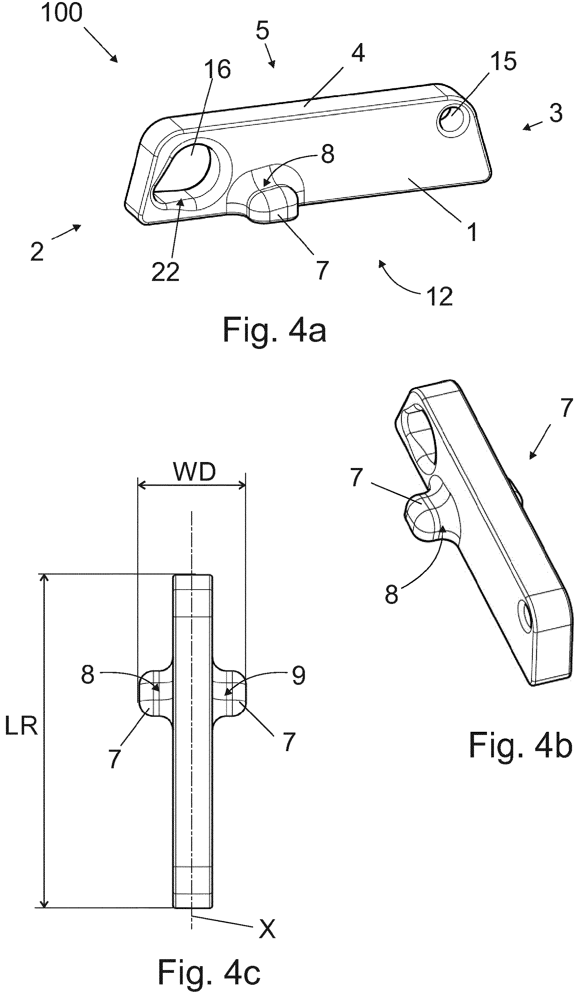

**Figures 4a - 4c** are schematic views of a fourth graft suspension device. In one embodiment, at least one of the first side routing surface 8 and the second side routing surface 9 is arranged on the outer surface of the projection 7. In the embodiment shown in Figures 4a-4c, both the first and second side routing surfaces 8, 9 are arranged on the outer surfaces of the projections 7.

It will be appreciated that, by having the holding surface 22 in the second through hole 22, a closed loop can be located in manufacturing of the device 100. Alternatively, the loop can be manufactured in situ.

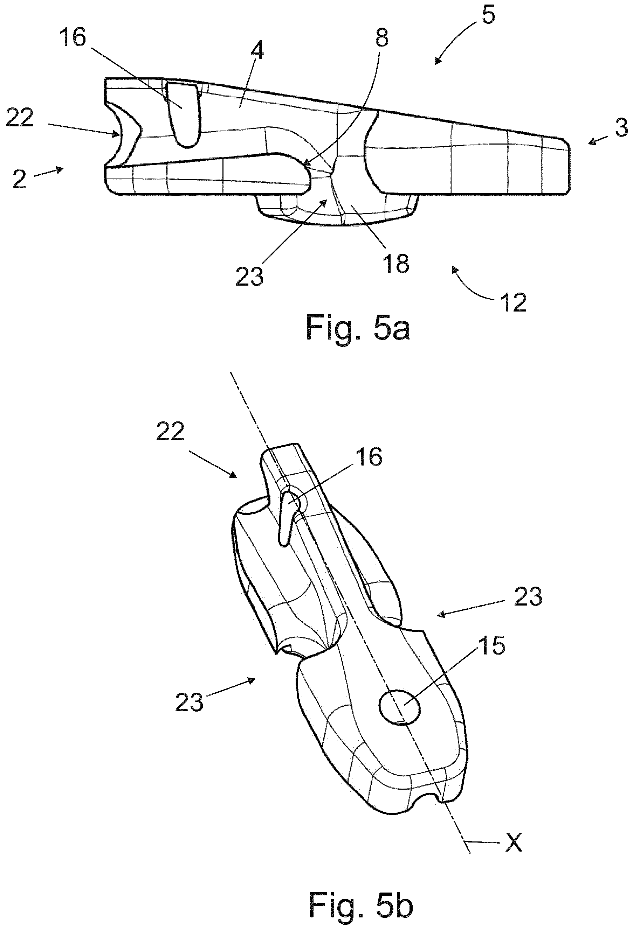

**Figures 5a, 5b** are schematic views of a fifth graft suspension device. In one embodiment, the holding surface 22 arranged for keeping the loop in place comprises a concavely shaped surface that is arranged closer to the first end 2 than to the second end 3 of the device. In some embodiments, such as shown in Figures 5a-5b, the holding surface 22 lies in the first end 2.

As already disclosed, the second through hole 16 may be arranged to extend from the first side 5 of the device to the second side 12 of the device. In some embodiments, such as shown in the previous Figures, the second through hole 16 is arranged symmetrically in relation to the longitudinal axis X of the device. In another embodiments, the second through hole 16 is asymmetrically arranged. One example of these embodiments is shown in Figures 5a, 5b, wherein the middle axis of the second through hole 16 lies left from the longitudinal axis X when the device is observed from the second end 3.

It will be appreciated that, by having open grooves 23 combined with the holding surface 22 arranged on the first end, a closed loop can be located after manufacture of the device 100. Alternatively, the loop can be manufactured in situ.

The graft suspension device 100 disclosed in this disclosure may be manufactured from various biocompatible materials.

In one embodiment, the material comprises metal material or alloy, such as stainless steel, titanium-based alloys, or magnesium-based alloys.

In one embodiment, the material comprises biostable polymer material and/or biodegradable polymer material. In one embodiment, the polymer material comprises reinforcing materials, such as fibres. An advantage of polymer materials is that they do not impair e.g. X-ray examination of the operated tissue.

In one embodiment, the polymer material is selected from polyetheretherketone PEEK, ultra-high-molecular-weight polyethylene UHMWPE, high-density polyethylene HDPE, poly(tetrafluoroethylene) PTFE, polymethyl methacrylate PMMA, polyethylene terephthalate PET, polypropylene PP, for instance.

In one embodiment, the polymer material is selected from polymer or copolymer of lactic acid, L-lactide, D-lactide, D,L-lactide, meso-lactide, glycolic acid, glycolide and the like and optionally other cyclic esters which are copolymerizable with lactide. Additional co-monomers may also be present to impart desired properties as needed such as alpha-, beta- or gamma-hydroxybutyric acid, alpha-, beta- or gamma-hydroxyvaleric acid and other hydroxy fatty acids (C11 to C25) such as stearic acid, palmitic acid, oleic acid, lauric acid and the like. Accordingly, base material include polylactides, polyglycolides, poly (L-lactide), poly (D-lactide), poly(L-lactide-co-D,L-lactide), poly(L-lactide-co-meso-lactide), poly(L-lactide-coglycolide), poly(L-lactide-co-epsilon-caprolactone), poly (D,L-lactide-co-meso-lactide), poly(D,L-lactide-co-glycolide), poly(D,L-lactide-co-epsilon-caprolactone), poly (meso-lactide-co-glycolide), poly(meso-lactide-co-epsilon-caprolactone) and the like. When the base material is a copolymer, the monomer units are present in a ratio of 50:50, 60:40, 70:30, 80:20, 85:15 and all suitable ratios in between. For example, suitable base materials include poly(L-lactide-co-D,L-lactide) 70:30, poly(L-lactide-co-D,L-lactide) 80:20, poly(L-lactide-co-glycolide) 85:15, and poly(L-lactide-co-glycolide) 80:20. Polymers or copolymers useful as base materials are commercially available from many sources or can be readily manufactured using methods well known to those skilled in the art.

**Figures 6a-6c** are schematic views of a graft suspension arrangement.

Viewed from a further aspect, there can be provided a graft suspension arrangement 200 that comprises the graft suspension device 100 as defined in this disclosure, a loop 19, a pulling suture 20 and optionally a flipping suture 21. Thereby a graft suspension arrangement that allows the use of lower strength materials than before may be achieved.

Figure 6a is showing the graft suspension arrangement 200 in a section of a channel 25 drilled in a bone tissue 26. The drilled channel may have 4.5 mm diameter, for instance.

The graft suspension arrangement 200 comprises the graft suspension device 100 described in this disclosure. In Figures 6a-6c the graft suspension device is similar to the device shown in Figures 2a-2c. In one embodiment, the graft suspension arrangement 200 further comprises a loop 19, a pulling suture 20 and a flipping suture 21.

The loop 19 is used for fixing a ligament (not shown) to the graft suspension device 100. In one embodiment, the loop 19 is of fiber material, for example suture material, such as polyethylene terephthalate PET, polypropylene PP, polyamide PA or expanded polytetrafluoroethylene ePTFE. In one embodiment, the loop 19 is manufactured from at least one bioabsorbable material. In another embodiment, the loop 19 is manufactured from at least one biostable material. In one embodiment, the loop 19 is manufactured from ultra-high-molecular-weight polyethylene UHMWPE.

Depending on the design of the graft suspension device 100, the loop 19 may be arranged after manufacture thereto, for example in situ. This kind of embodiments are shown in Figures 1a-1c, 2a-2c and 5a-5b. In some other embodiments, the loop 19 may be arranged in the device 100 during manufacturing thereof. Alternatively, the loop can be manufactured (closed) in situ.

The pulling suture 20 is passed through the second through hole 16 and also through the loop 19. As the arrangement 200 is drawn through the channel 25 in direction P by using the pulling suture 20, the pulling suture 20 presses the loop 19 against the plateau 6.

The flipping suture 21 is passed through the first through hole 15 and directed by the device 100 to the same direction as the pulling suture 20 in the channel 25. The flipping suture 21 is also drawn in direction P together with the pulling suture 20.

In another embodiment, the pulling suture 20 is passed through the first through hole 15 and the flipping suture 21 is passed through the second through hole 16, i.e. vice versa compared to Figures 6a, 6b. The device 100 is then arranged in the channel 25 so that the sutures 20, 21 as well as the loop 19 are directed in opposite directions as shown in Figures 6a, 6b, and the device 100 is drawn through the channel 25 in direction opposite to the direction P shown in Figure 6a. It is to be noted here, that the device 100 described in this disclosure can be arranged in and drawn through the channel 25 two alternative directions, i.e. they can be drawn either the first end 2 or the second end 3 as a leading end through the channel.

Figure 6c is showing the graft suspension arrangement 200 in a securing position where the the graft suspension device 100 has been rotated flat across the channel 25. This position may be achieved as soon as the device 100 exits from the channel 25 and has room to be rotated by the flipping suture 21. As the correct position achieved, the pulling suture 20 and the flipping suture 21 are drawn away from the device 100, and the suture loop 19 is allowed to move onto the ridge 4.

In some embodiments, the loop 19 is on a holding surface 22 arranged in a hole. Some of these embodiments are shown in Figures 3a-3b and 4a-4c. As this kind of device 100 is in the securing position, the loop 19 stays in said hole and runs along the first and the second side routing surfaces 8, 9 in the channel 25.

In some embodiments, the loop 19 is arranged on a holding surface 22 that comprises a concavely shaped surface. One embodiment of this kind is shown in Figures 5a-5b. As this kind of device 100 is in the securing position, the loop 19 stays on the holding surface and runs along the first and the second side routing surfaces 8, 9 in the channel 25.

Figure 6c is showing how the ridge 4 is so long that it extends over the channel 25 and creates a load carrying beam over the channel. In one embodiment, the presence of such load carrying beam is guaranteed by dimensioning the length LR of the ridge to exceed the width WD of the device.

If the device comprises the middle bulge 18, such as shown in Figures 2a-2c and 5a, it preferably protrudes in the channel 25 as shown in Figure 6c. This strengthens the load carrying beam even further.

It will be appreciated that the device 100 may be used during knee surgery for fixation of a graft, wherein the graft may include a ligament or tendon being either a transplant or artificial.

The drawings and the related description are only intended to illustrate the idea of the invention. The invention may vary in detail within the scope of the inventive idea defined in the following claims.

### REFERENCE SYMBOLS

- 1: body
- 2: first end
- 3: second end
- 4: ridge
- 5: first side
- 6: plateau
- 7: projection
- 8: first side routing surface
- 9: second side routing surface
- 10: first sidewall
- 11: second sidewall
- 12: second side
- 13: sloping surface
- 14: top
- 15: first through hole
- 16: second through hole
- 17: end wall
- 18: middle bulge
- 19: loop
- 20: pulling suture
- 21: flipping suture
- 22: holding surface
- 23: groove
- 24: hole
- 25: drilled channel
- 26: bone tissue
- 100: graft suspension device
- 200: graft suspension arrangement

- HD: height of the device
- P: pulling
- X: longitudinal axis
- LR: length of the ridge
- WD: width of the device

## Claims

1. A graft suspension device (100), comprising
- an elongate body (1) having a first end (2), a second end (3), and a longitudinal axis (X) extending therebetween,
- a ridge (4) arranged on the longitudinal axis, on a first side (5) of the graft suspension device,
- a first side routing surface (8) arranged next to a first side of the ridge,
- a second side routing surface (9) arranged next to a second side of the ridge, **characterized in that**
- the ridge (4) lies between the first and second routing surfaces (8, 9) when seeing from direction of the first side (5), and wherein
- the first and second routing surfaces (8, 9) are extending from the first side (5) to a second side (12) of the device, and that
- at least one of the first side routing surface (8) and the second side routing surface (9) is arranged in a groove (23) that is arranged to constitute a part of a respective sidewall (10, 11) of the device, or that
- at least one of the first side routing surface (8) and the second side routing surface (9) is arranged in a hole (24).

2. The device as claimed in claim 1, comprising
- at least one projection (7) extending sideways away from the ridge, and
- wherein at least one of the first side routing surface (8) and the second side routing surface (9) is arranged on the surface of the at least one projection (7).

3. The device as claimed in any of the preceding claims, wherein
- the side routing surfaces (8, 9) are arranged asymmetrically so that one of the side routing surfaces is located closer to the first end (2) than another side routing surface.

4. The device as claimed in any of the preceding claims, comprising
- a first through hole (15) extending through the device and
- arranged closer to the second end (3) than to the first end (2).

5. The device as claimed in any of the preceding claims, comprising
- a second through hole (16) extending through the device and
- arranged closer to the first end (2) than to the second end (3).

6. The device as claimed in any of the preceding claims, comprising
- a holding surface (22) comprising a concave shape, the holding surface(22) being arranged closer to the first end (2) than to the second end (3).

7. The device as claimed in claim 5 or 6, comprising
- a holding surface (22) comprising a concave shape, and wherein
- the holding surface (22) is arranged in the second through hole (16).

8. The device as claimed in any of claims 6 - 7, comprising
- a plateau (6) arranged between the ridge (4) and the first end (2) on the first side (5) of the graft suspension device,
- the plateau (6) joining to the side routing surfaces (8, 9), and wherein
- the second through hole (16) is arranged in the plateau (6).

9. The device as claimed in claim 8, comprising
- an end wall (17) arranged at the first end (2), the end wall (17) bordering the plateau (6).

10. The device as claimed in any of claims 8 - 9, wherein
- the ridge (4) has a sloping surface (13) from the plateau (6) on the ridge.

11. The device as claimed in any of the preceding claims, comprising
- a middle bulge (18) arranged in an area between the first and the second side routing surfaces (8, 9) on the second side (12) of the device.

12. The device as claimed in any of the preceding claims,
- being made of at least one of the materials selected from polymer materials and metals.

13. A graft suspension arrangement (200), comprising:
- the graft suspension device (100) as claimed in any of the preceding claims,
- a loop (19),
- a pulling suture (20), and optionally
- a flipping suture (21).

## Patentansprüche

1. Transplantataufhängungsgerät (100), umfassend
- einen länglichen Körper (1) mit einem ersten Ende (2), einem zweiten Ende (3) und einer sich dazwischen erstreckenden Längsachse (X),
- eine Erhöhung (4), die auf der Längsachse auf einer ersten Seite (5) des Transplantataufhängungsgeräts eingerichtet ist,
- eine erste Seitenführungsoberfläche (8), die neben einer ersten Seite der Erhöhung eingerichtet ist,
- eine zweite Seitenführungsoberfläche (9), die neben einer zweiten Seite der Erhöhung eingerichtet ist, **dadurch gekennzeichnet, dass**
- die Erhöhung (4) zwischen der ersten und der zweiten Führungsoberfläche (8, 9) liegt, wenn sie aus der Richtung der ersten Seite (5) betrachtet wird, und wobei
- sich die erste und die zweite Führungsoberfläche (8, 9) von der ersten Seite (5) zu einer zweiten Seite (12) des Geräts erstrecken, und dass
- mindestens eine der ersten Seitenführungsoberfläche (8) und der zweiten Seitenführungsoberfläche (9) in einer Nut (23) eingerichtet ist, die dazu eingerichtet ist, einen Teil einer entsprechenden Seitenwand (10, 11) des Geräts darzustellen, oder dass
- mindestens eine der ersten Seitenführungsoberfläche (8) und der zweiten Seitenführungsoberfläche (9) in einem Loch (24) eingerichtet ist.

2. Gerät nach Anspruch 1, umfassend
- mindestens einen Vorsprung (7), der sich seitlich von der Erhöhung weg erstreckt, und
- wobei mindestens eine der ersten Seitenführungsoberfläche (8) und der zweiten Seitenführungsoberfläche (9) auf der Oberfläche des mindestens einen Vorsprungs (7) eingerichtet ist.

3. Gerät nach einem der vorangehenden Ansprüche, wobei
- die Seitenführungsoberflächen (8, 9) asymmetrisch eingerichtet sind, so dass sich eine der Seitenführungsoberflächen näher an dem ersten Ende (2) befindet als eine andere Seitenführungsoberfläche.

4. Gerät nach einem der vorangehenden Ansprüche, umfassend
- ein erstes Durchgangsloch (15), das sich durch das Gerät erstreckt und
- näher an dem zweiten Ende (3) als an dem ersten Ende (2) eingerichtet ist.

5. Gerät nach einem der vorangehenden Ansprüche, umfassend
- ein zweites Durchgangsloch (16), das sich durch das Gerät erstreckt und
- näher an dem ersten Ende (2) als an dem zweiten Ende (3) eingerichtet ist.

6. Gerät nach einem der vorangehenden Ansprüche, umfassend
- eine Halteoberfläche (22), die eine konkave Form umfasst, wobei die Halteoberfläche (22) näher an dem ersten Ende (2) als an dem zweiten Ende (3) eingerichtet ist.

7. Gerät nach Anspruch 5 oder 6, umfassend
- eine Halteoberfläche (22), die eine konkave Form umfasst, und wobei
- die Halteoberfläche (22) in dem zweiten Durchgangsloch (16) eingerichtet ist.

8. Gerät nach einem der Ansprüche 6 - 7, umfassend
- ein Plateau (6), das zwischen der Erhöhung (4) und dem ersten Ende (2) auf der ersten Seite (5) des Transplantataufhängungsgeräts eingerichtet ist,
- wobei das Plateau (6) mit den Seitenführungsoberflächen (8, 9) verbunden ist, und wobei
- das zweite Durchgangsloch (16) in dem Plateau (6) eingerichtet ist.

9. Gerät nach Anspruch 8, umfassend
- eine Endwand (17), die an dem ersten Ende (2) eingerichtet ist, wobei die Endwand (17) an das Plateau (6) angrenzt.

10. Gerät nach einem der Ansprüche 8 - 9, wobei
- die Erhöhung (4) eine geneigte Oberfläche (13) von dem Plateau (6) auf der Erhöhung aufweist.

11. Gerät nach einem der vorangehenden Ansprüche, umfassend
- eine mittlere Wölbung (18), die in einem Gebiet zwischen der ersten und der zweiten Seitenführungsoberfläche (8, 9) auf der zweiten Seite (12) des Geräts eingerichtet ist.

12. Gerät nach einem der vorangehenden Ansprüche,
- das aus mindestens einem der Materialien hergestellt ist, die aus Polymermaterialien und Metallen ausgewählt sind.

13. Transplantataufhängungsanordnung (200), umfassend:
- das Transplantataufhängungsgerät (100) nach einem der vorangehenden Ansprüche,
- eine Schlaufe (19),
- einen Zugfaden (20), und optional
- einen Kippfaden (21).

## Revendications

1. Dispositif de suspension de greffe (100), comprenant
- un corps allongé (1) présentant une première extrémité (2), une seconde extrémité (3) et un axe longitudinal (X) s'étendant entre celles-ci,
- une crête (4) disposée sur l'axe longitudinal, sur un premier côté (5) du dispositif de suspension de greffe,
- une première surface de guidage latérale (8) disposée près d'un premier côté de la crête,
- une seconde surface de guidage latérale (9) disposée près d'un second côté de la crête, **caractérisé en ce que**
- la crête (4) se situe entre les première et seconde surfaces de guidage (8, 9) lorsqu'on regarde depuis la direction du premier côté (5), et dans lequel
- les première et seconde surfaces de guidage (8, 9) s'étendent depuis le premier côté (5) jusqu'à un second côté (12) du dispositif, et **en ce que**
- au moins l'une parmi la première surface de guidage latérale (8) et la seconde surface de guidage latérale (9) est disposée dans une rainure (23) qui est disposée pour constituer une partie d'une paroi latérale respective (10, 11) du dispositif, ou **en ce que**
- au moins l'une parmi la première surface de guidage latérale (8) et la seconde surface de guidage latérale (9) est disposée dans un trou (24).

2. Dispositif selon la revendication 1, comprenant
- au moins une saillie (7) s'étendant latéralement à partir de la crête, et
- dans lequel au moins l'une parmi la première surface de guidage latérale (8) et la seconde surface de guidage latérale (9) est disposée sur la surface de l'au moins une saillie (7).

3. Dispositif selon l'une quelconque des revendications précédentes, dans lequel
- les surfaces de guidage latérales (8, 9) sont disposées de manière asymétrique de sorte que l'une des surfaces de guidage latérales est située plus près de la première extrémité (2) que l'autre surface de guidage latérale.

4. Dispositif selon l'une quelconque des revendications précédentes, comprenant
- un premier trou traversant (15) s'étendant à travers le dispositif et
- disposé plus près de la seconde extrémité (3) que de la première extrémité (2).

5. Dispositif selon l'une quelconque des revendications précédentes, comprenant
- un second trou traversant (16) s'étendant à travers le dispositif et
- disposé plus près de la première extrémité (2) que de la seconde extrémité (3).

6. Dispositif selon l'une quelconque des revendications précédentes, comprenant
- une surface de maintien (22) comprenant une forme concave, la surface de maintien (22) étant disposée plus près de la première extrémité (2) que de la seconde extrémité (3).

7. Dispositif selon la revendication 5 ou 6, comprenant
- une surface de maintien (22) comprenant une forme concave, et dans lequel
- la surface de maintien (22) est agencée dans le second trou traversant (16).

8. Dispositif selon l'une quelconque des revendications 6 et 7, comprenant
- un plateau (6) disposé entre la crête (4) et la première extrémité (2) sur le premier côté (5) du dispositif de suspension de greffe,
- le plateau (6) s'unissant aux surfaces de guidage latérales (8, 9), et dans lequel
- le second trou traversant (16) est disposé dans le plateau (6).

9. Dispositif selon la revendication 8, comprenant
- une paroi d'extrémité (17) disposée au niveau de la première extrémité (2), la paroi d'extrémité (17) bordant le plateau (6).

10. Dispositif selon l'une quelconque des revendications 8 et 9, dans lequel
- la crête (4) présente une surface inclinée (13) à partir du plateau (6) sur la crête.

11. Dispositif selon l'une quelconque des revendications précédentes, comprenant
- un renflement médian (18) disposé dans une zone entre les première et seconde surfaces de guidage latérales (8, 9) sur le second côté (12) du dispositif.

12. Dispositif selon l'une quelconque des revendications précédentes,
- qui est constitué d'au moins un des matériaux sélectionnés à partir des matériaux polymères et des métaux.

13. Agencement de suspension de greffe (200), comprenant :
- le dispositif de suspension de greffe (100) selon l'une quelconque des revendications précédentes,
- une boucle (19),
- une suture de traction (20) et, facultativement,
- une suture à retournement (21).
